## Europäisches Patentamt
## European Patent Office
## Office européen des brevets

(19)

(11) Veröffentlichungsnummer: **0 009 148**
**B1**

(12) **EUROPÄISCHE PATENTSCHRIFT**

(45) Veröffentlichungstag der Patentschrift:
**02.03.83**

(21) Anmeldenummer: **79103210.5**

(22) Anmeldetag: **30.08.79**

(51) Int. Cl.³: **A 61 F 1/03, C 04 B 35/52,
C 01 B 31/36**

(54) **Gelenkendoprothese.**

(30) Priorität: **02.09.78 DE 2838333
02.09.78 DE 2838335**

(43) Veröffentlichungstag der Anmeldung:
**02.04.80 Patentblatt 80/7**

(45) Bekanntmachung des Hinweises auf die Patenterteilung:
**02.03.83 Patentblatt 83/9**

(84) Benannte Vertragsstaaten:
**AT CH DE FR GB IT SE**

(56) Entgegenhaltungen:
**DE-A-2 707 299
DE-B-832
DE-B-1 796 279
DE-B-2 718 142
US-A-3 685 059
US-A-3 707 006
US-A-3 877 080**

**Die Akte enthält technische Angaben, die nach dem
Eingang der Anmeldung eingereicht wurden und die
nicht in dieser Patentschrift enthalten sind.**

(73) Patentinhaber: **Schunk & Ebe GmbH, Rodheimer
Strasse 59-61, D-6301 Heuchelheim (DE)**

(72) Erfinder: **Hüttinger, Klaus J., Prof. Dr., Allensteiner
Strasse 17, D-7500 Karlsruhe (DE)**
Erfinder: **Rosenblatt, Ulf, Dr., Dipl.-Chem., Amselweg 1,
D-6300 Lahn-Heuchelheim 1 (DE)**
Erfinder: **Brückmann, Heinz, Dipl.-Phys.,
Eichendorffring 72, D-6300 Lahn-Giessen 1 (DE)**
Erfinder: **Busche, Claus, Dorfstrasse 51,
D-6331 Rechtenbach 2 (DE)**

(74) Vertreter: **Hann, Michael, Dr., Marburger Strasse 38,
D-6300 Giessen (DE)**

BUNDESDRUCKEREI BERLIN

Gelenkendoprothese

Die Erfindung betrifft eine Gelenkendoprothese mit zwei aufeinander gleitenden Prothesenteilen.

Der Ersatz von natürlichen Gelenken, die ihre Funktionsfähigkeit durch Krankheit oder Unfall verloren haben, durch künstliche Gelenke ist bekannt. Solche Gelenke sind u. a. das Hüft-, Knie-, Sprung-, Ellbogen-, Schulter- oder Fingergelenk. Bestimmend für die Funktionstüchtigkeit einer Gelenkendoprothese ist einerseits die Gleitpaarung des Gelenkes und andererseits die Verankerung im natürlichen Knochen. Es ist außer Frage, daß für die Gleitpaarungen des Gelenkes und für das Verankerungsteil jeweils Werkstoffe mit sehr spezifischen Werkstoffeigenschaften erforderlich sind.

Von den Gleitpaarungen sind hervorragende tribologische Eigenschaften und extrem niedere Verschleißraten unter den besonderen Beanspruchungen im Körper und eine vollständige Biokompatibilität des Abriebes zu fordern. Für die Verankerungsteile ist in erster Linie die mechanische Kompatibilität mit dem knöchernen Lager entscheidend. Als Werkstoffkombination für die Gleitpaarung wird derzeit überwiegend Metall mit höchstmolekularem Niederdruck-Polyäthylen verwendet. Diese Gleitpaarung hat sich in begrenztem Umfang bewährt.

Die derzeit bekannte, mittlere Funktionstüchtigkeit von Gelenkendoprothesen liegt entscheidend unter 10 Jahren, was im Sinne eines dauerhaften Gelenkersatzes als unbefriedigend zu bezeichnen ist.

Wenn sich die beschränkte Funktionstüchtigkeit auch nicht eindeutig der Gleitpaarung beziehungsweise dem Verankerungsteil zuordnen läßt, so steht fest, daß das Polyäthylen infolge der hohen mechanischen Belastung des Gelenkes zum Kaltfluß neigt und darüber hinaus Alterungsprozessen unterworfen ist, welche die mechanischen und tribologischen Eigenschaften verschlechtern.

Außerdem haben die entstehenden Abriebpartikel, sowohl des Polyäthylens, als auch der Metalle beziehungsweise Metallegierungen, negative biologische Reaktionen zur Folge. Schließlich ist zu erwähnen, daß Verschleiß und Kaltfluß des Polyäthylens bei fortgeschrittenem Stadium zu einer Funktionsminderung der Prothesen durch die sich mit der Zeit ändernden relativen geometrischen Positionen der Gleitpartner zueinander führen.

Es ist deshalb in der DE-A-2 700 621 vorgeschlagen worden, das Polyäthylen mit Kurzschnittfasern zu verstärken. Durch diese Maßnahme können die Neigung des Polyäthylens zum Kaltfluß und die Abriebrate verringert werden. Befriedigende Funktionsdauern können aber dennoch nicht erreicht werden, da durch die Faserverstärkung die Alterungserscheinungen der Polyäthylenmatrix und die damit verbundenen Konsequenzen, wie erhöhte Reibung und erhöhter Verschleiß, nicht beeinflußt werden.

Aus der DE-C-2 134 316 ist bekannt, Kugel und Pfanne von Hüftgelenkendoprothesen aus $Al_2O_3$ zu fertigen. Mit dieser Gleitpaarung werden die erstrebten niedrigen Verschleißraten erreicht, solange keine Trockenreibung durch zu hohe Flächenpressung vorliegt.

Unabhängig davon hat auch diese Gleitpaarung ihre besonderen Nachteile. Es kann erstens wegen des extrem hohen Elastizitätsmoduls der $Al_2O_3$-Keramik keine mechanische Kompatibilität mit dem knöchernen Lager erreicht werden; zweitens sind wegen der komplizierten Formen der menschlichen Gelenkflächen bei Gleitwerkstoffen mit sehr hohen Elastizitätsmoduli linienbeziehungsweise punktförmige Berührungen nicht immer zu vermeiden, deren Folge untragbare Verschleißraten sind.

Es ist auch eine Prothese mit einer Gleitpaarung aus $Al_2O_3$ und Polyäthylen bekannt. Diese Gleitpaarung unterliegt jedoch nach wie vor den Nachteilen des Polyäthylens, wie Kaltfluß, Alterung und zu hohe Verschleißraten.

Man hat auch schon vorgeschlagen, Siliziumcarbid/Kohlenstoff (SiC/C)-Werkstoffe, die durch Gasphasenpyrolyse erhalten worden sind, für die Herstellung von Gelenkendoprothesen zu verwenden. Derartige Prothesen sind z. B. in der US-A-3 877 080 beschrieben. Zur Herstellung solcher SiC/C-Werkstoffe werden leicht flüchtige Kohlenwasserstoffe und leicht flüchtige Siliziumverbindungen, wie Methyltrichlorsilan, in der Gasphase auf einem erhitzten Substrat pyrolisiert. Dabei werden das Siliziumcarbid und der Kohlenstoff gleichzeitig auf dem Substrat abgeschieden. Die beiden Phasen SiC und C sind mikrodispers in dem erhaltenen Werkstoff verteilt. Prothesenteile aus einem derartigen mikrodispersen SiC/C-Werkstoff haben nur unbefriedigende Gleiteigenschaften.

Der Erfindung liegt deshalb die Aufgabe zugrunde, eine Gelenkendoprothese der eingangs genannten Art zu schaffen, die die oben beschriebenen Nachteile herkömmlicher Gelenkendoprothesen nicht aufweist und insbesondere folgenden Anforderungen in optimaler Weise Rechnung trägt.

1. Absolute biologische Kompatibilität
   Diese Forderung besagt, daß der Implantatwerkstoff weder in Form des Implantates noch in Form des durch die Reibung entstehenden Abriebstaubes irgendeine Abstoßungs- oder Entzündungsreaktion des Körpergewebes hervorrufen darf.
2. Optimale Gleit- und Verschleißeigenschaften
   Diese Forderung besagt, daß der Reibungskoeffizient möglichst niedrig sein muß, um die durch Reibung übertragenen Momente auf das jeweilige Gegenlager und damit auf die Grenzschicht Implantat/Knochen so gering wie möglich zu halten.

Die Verschleißraten müssen so niedrig sein, daß die Menge des Abriebstaubes vom Gewebe toleriert wird und die Eindringtiefe des einen Lagers in das Gegenlager auch nach jahrzehntelangem Gebrauch so gering ist, daß keine Funktionsminderung durch Einschränkung des Bewegungsumfanges resultiert.

3.    Mechanische Kompatibilität

Diese Forderung besagt, daß der Kraftflußlauf vom Prothesenlager in das knöcherne Gegenlager sich am physiologischen Kraftfluß in den knöchernen Strukturen orientieren muß. Große Steifigkeitssprünge zwischen Implantat und Knochen bewirken örtliche Spannungsspitzen, die den Knochen zu Abbaureaktionen veranlassen und damit Lockerungen hervorrufen. Es muß deshalb an ein Implantatmaterial die Forderung gestellt werden, daß der Elastizitätsmodul größenordnungsmäßig dem Elastizitätsmodul von kompakten Knochenmaterialien entspricht.

Diese Aufgabe wird durch eine Gelenkendoprothese mit zwei aufeinander gleitenden Prothesenteilen gelöst, bei der mindestens die Gleitfläche eines aus polykristallinem Kohlenstoff vorgefertigten Prothesenteils aus einem feinverteiltes Siliziumcarbid enthaltendem Kohlenstoffwerkstoff besteht, wobei diese Gelenkendoprothese dadurch gekennzeichnet ist, daß der Siliziumcarbid enthaltende Kohlenstoffwerkstoff erhalten wurde durch Imprägnieren des vorgefertigten Prothesenteils mit geschmolzenem Silizium.

Das durch Imprägnieren eines vorgefertigten Prothesenteils aus polykristallinem Kohlenstoff mit geschmolzenem Silizium erhaltene Material wird nachstehend kurz auch als »SiC/C-Werkstoff« bezeichnet. In der Regel besteht der SiC/C-Werkstoff bei dem Prothesenteil gemäß der Erfindung aus 30 bis 70 Gew.-% Kohlenstoff und 70 bis 30 Gew.-% Siliziumcarbid.

Der Kohlenstoff und das Siliziumcarbid liegen in dem SiC/C-Werkstoff bevorzugt in feinverteilter Form vor, wobei die größten Teilchen in der Regel eine maximale Abmessung von 200 µm haben. Das Siliziumcarbid bildet weitgehend eine zusammenhängende Matrix, in die einzelne Kohlenstoffkörner eingelagert sind. Im Regelfall sind die kleinsten Kohlenstoffteilchen nicht kleiner als 5 µm, und ein besonders bevorzugter Bereich der Teilchengrößen liegt bei 10 bis 60 µm.

Die Imprägnierung des vorgefertigten Prothesenteils aus polykristallinem Kohlenstoff mit geschmolzenem Silizium wird wegen der hohen Temperaturen und der Oxidierbarkeit der Ausgangsstoffe in Gegenwart eines inerten oder reduzierenden Gases durchgeführt.

Es hat sich als besonders vorteilhaft erwiesen, für die Gleitfläche des anderen Prothesenteils eines Gelenkes bzw. für das gesamte Prothesenteil ebenfalls den durch Imprägnieren mit geschmolzenem Silizium erhaltenen SiC/C-Werkstoff zu verwenden. In anderen Fällen kann es jedoch auch günstig sein, für das andere Prothesenteil als Werkstoff Kohlenstoff, insbesondere isotropen Kohlenstoff, einzusetzen. Unter isotropem Kohlenstoff ist ein feinkörniger, polykristalliner Kohlenstoff zu verstehen, der aus kohlenstoffhaltigen Feststoffen und einem organischen Bindemittel nach keramischen Arbeitsmethoden herstellbar ist. Vorteilhaft hat dieser Kohlenstoffwerkstoff eine Härte von >35 HB 2,5/5 und eine Biegefestigkeit von >70 N/mm².

Die überragenden Eigenschaften des durch Imprägnieren mit geschmolzenem Silizium hergestellten SiC/C-Prothesenteils kommen jedoch in einer Pothese auch dann noch zur Wirkung, wenn das andere Prothesenteil aus bekannten Materialien, wie Metall oder Polyäthylen, besteht, so daß ein solches Prothesenteil auch in dieser Kombination verwendet werden kann.

Das Prothesenteil einer erfindungsgemäßen Gelenkendoprothese kann aus mehreren Gliedern bestehen, wobei mindestens die Gleitfläche des Prothesenteils von dem Siliziumcarbid-Kohlenstoff-Werkstoff, der durch Imprägnieren von polykristallinem Kohlenstoff mit geschmolzenem Silizium erhalten wurde, gebildet wird. Die einzelnen Glieder des Prothesenteils können in der Weise miteinander verbunden worden sein, daß mehrere vorgefertigte Glieder aus Kohlenstoffbasiswerkstoffen formschlüssig angepaßt und mit Silizium unter Erwärmen behandelt worden sind. Gleichzeitig mit der Ausbildung des Siliziumcarbid-Kohlenstoff-Werkstoffes an der Gleitfläche entsteht dabei an den Berührungsflächen der einzelnen Glieder eine Siliziumcarbid-Verbindungsnaht, durch die die einzelnen Glieder kraftschlüssig miteinander verbunden werden.

Bevorzugt ist ein derartiges aus mehreren Gliedern bestehendes Prothesenteil dadurch erhältlich, daß die vorgefertigten, formschlüssigen Prothesenglieder in einem Vakuum-Druck-Verfahren in einer flüssigen Siliziumschmelze unter Reaktion des Siliziums mit Kohlenstoff aus den Prothesengliedern. Diese Verbindungsschicht besitzt eine Scherfestigkeit von der gleichen Größenordnung wie diejenige der zu verbindenden Teile.

Die Gelenkendoprothese nach der Erfindung kann auch ein Prothesenteil aus mehreren Gliedern besitzen, bei dem ein Glied eine Gleitfläche aus mit geschmolzenem Silizium imprägniertem polykristallinem Kohlenstoff besitzt, und dieses Glied und andere vorgefertigte, formschlüssige Prothesenglieder an den Verbindungsflächen oberflächlich mit einer Aufschlämmung von Siliziumpulver bestrichen worden sind. Die so behandelten Prothesenglieder sind zusammengefügt, getrocknet und zur Ausbildung einer Siliziumcarbid-Verbindungsschicht durch Reaktion des Siliziums mit Kohlenstoff auf den Prothesengliedern behandelt worden. Die Wärmebehandlung zum Verbinden der Glieder kann zum Beispiel bei etwa 2000° C stattfinden.

Das in den erfindungsgemäßen Gelenkendoprothesen vorgesehene Prothesenteil aus dem SiC/C-Werkstoff weist den Vorteil auf, daß weder das Siliziumcarbid noch der Kohlenstoff noch die Kombination der beiden Werkstoffkomponenten irgendeine Abstoßungsreaktion des Körpers verursachen. Dies gilt sowohl für kompakte Prothesenteile als auch für die im künstlichen Gelenk entstehenden Abriebpartikel.

Die überragende Biokompatibilität dieses SiC/C-Werkstoffes hat weiter zur Folge, daß bei Verankerung eines Prothesenteils im Knochen der Knochen formschlüssig an das Implantat heranwächst. Somit ist es mit diesem Verbundwerkstoff auch möglich, auf den üblicherweise zur Fixierung verwendeten Knochenzement zu verzichten. Solche Anwendungen sind beispielsweise gegeben bei der Fixierung von Hüftgelenkspfannen oder Kappenprothesen für den Hüftgelenkskopf.

Das höchstbelastete menschliche Gelenk, nämlich das Hüftgelenk, ist bei relativen Gleitgeschwindigkeiten von etwa 0,05 m/sec mit maximalen Lasten von etwa 3.000 N beaufschlagt. Für künstliche Hüftgelenke, deren Gleitpartner ausschließlich aus dem erfindungsgemäßen Verbundwerkstoff bestehen, ergeben sich maximale Flächenpressungen von etwa 20 N/mm². In diesem Belastungsbereich besitzt der Reibungskoeffizient den außergewöhnlich niedrigen Wert von ca. 0,1.

Die überragenden Eigenschaften eines Prothesenteils mit dem SiC/C-Werkstoff zeigen sich teilweise auch bei Verwendung von isotropem Kohlenstoff der eingangs beschriebenen Art als Gleitpartner. Diese Einschränkung bezieht sich ausschließlich auf die Biegefestigkeit des isotropen Kohlenstoffs, in keinem Falle jedoch auf die Biokompatibilität. Für eine Gleitpaarung aus dem Verbundwerkstoff mit dem isotropen Kohlenstoff für das Hüftgelenk ergibt sich, entsprechend den voranstehend beschriebenen Belastungen, eine maximale Flächenpressung von nur 10 N/mm².

Diese Reduzierung der maximalen Flächenpressung gegenüber einer Gleitpaarung, bei der beide Gleitpartner aus dem SiC/C-Werkstoff bestehen, ist auf den kleineren Druckmodul des isotropen Kohlenstoffs zurückzuführen. Aber selbst für diese Gleitpaarung mit dem weicheren, isotropen Kohlenstoff betragen die jährlichen Verschleißraten bei mittlerer Belastung des Hüftgelenks weniger als 0,04 mm³. Diese verschwindend geringe Menge wird unter Berücksichtigung der Biokompatibilität der Werkstoffkomponenten in jedem Falle vom Körpergewebe toleriert. Die Eindringtiefe des Kopfes in die Pfanne von etwa 2 bis 4 µm pro Jahr liegt um zwei Zehnerpotenzen niedriger als bei der herkömmlichen Gleitpaarung Metall/Polyäthylen. Bei einer Beanspruchungsdauer des künstlichen Gelenkes von 30 Jahren resultieren für die Gleitpaarung SiC/C-Werkstoff/isotroper Kohlenstoff Eindringtiefen von ca. 0,12 mm. Solche Eindringtiefen führen in keinem Falle zu einer Verminderung der Funktionsfähigkeit infolge verminderten Bewegungsumfangs.

Für die herkömmliche Gleitpaarung Metall/Polyäthylen ergibt sich dagegen nach 30 Jahren eine rechnerische Verschleißstufe des Polyäthylens von etwa 10 mm. Verschleißtiefen von mehreren Millimetern führen zu Einschränkungen des Bewegungsumfangs und zu Hebelwirkungen vom Prothesenhals auf den Prothesenrand. Daraus wiederum resultieren unzulässige Belastungen an der Grenzfläche Knochen/Implantat. In der Praxis werden Eindringtiefen von 10 mm nicht beobachtet, da die Prothesen infolge Lockerung in der Regel bereits nach 7 bis 10 Jahren explantiert werden müssen.

Ein weiterer Vorteil der Gleitpaarungen, bei denen ein Gleitpartner eine Gleitfläche aus dem SiC/C-Werkstoff besitzt, liegt in der bekannten Tatsache, daß Kohlenstoffwerkstoffe Notlaufeigenschaften besitzen, die dann wesentlich sind, wenn durch Fehlstellung oder kurzzeitige Überlastung an Prothesengleitflächen derart hohe Flächenpressungen entstehen, daß der Schmierfilm abreißt. In solchen Fällen zeigen die konventionellen Gleitwerkstoffe ein katastrophales Ansteigen der Verschleißraten. Verschleißvorgänge dieser Art sind bei Gleitpaarungen mit dem Verbundwerkstoff als Gleitpartner nicht zu erwarten, weil Kohlenstoff infolge seiner lamellaren Kristallstruktur auch bei Trockenreibung gute Gleiteigenschaften aufweist.

Betrachtet man die Gleitpaarung des Verbundwerkstoffes mit einem metallischen Gleitpartner, so liegt der Vorteil einer derartigen Gleitpaarung in dem Ersatz des bisher allgemein verwendeten Polyäthylens und damit in der Vermeidung der eingangs aufgeführten Nachteile des Polyäthylens. Als konkretes Beispiel sei auf die Möglichkeit des Ersatzes des Tibia-Plateaus durch den SiC/C-Werkstoff anstelle von Polyäthylen hingewiesen.

Ein weiterer Vorteil der erfindungsgemäßen Gelenkendoprothese ist in der geringen Dichte des SiC/C-Werkstoffes von etwa 2,6 g/cm³ zu sehen. Bei der noch geringeren Dichte des als günstiger Gleitpartner in Frage kommenden isotropen Kohlenstoffes von 1,8 g/cm³ ergeben sich dadurch bei den verhältnismäßig voluminösen künstlichen Hüft- und Kniegelenken beachtliche Gewichtsersparnisse gegenüber Metallprothesen.

Die Erfindung wird in den Beispielen noch näher erläutert.

Beispiel 1

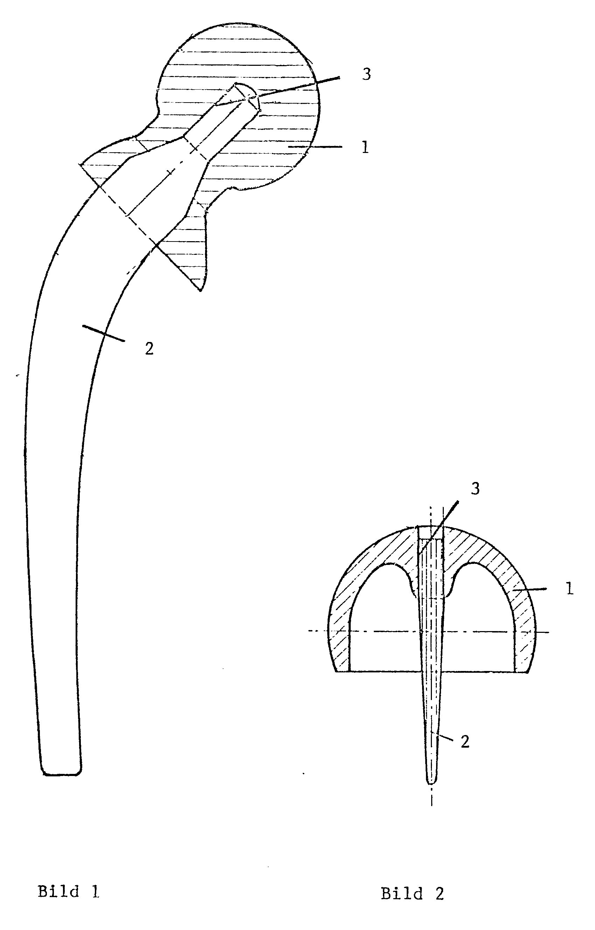

Bild 1 zeigt eine Kappenprothese mit Stift für künstliche Hüftgelenke. Bei dieser Prothese besteht die Kappe 1 aus einem Siliziumcarbid-Kohlenstoff-Verbundwerkstoff und der die Verankerung unterstützende Stift 2 aus einem kohlenstoff-faserverstärkten Kohlenstoff. Eine solche Prothese wird in folgenden Schritten hergestellt:

(1) Fertigung der Kappe aus polykristallinem Kohlenstoff;
(2) Fertigung des Stiftes aus kohlenstoffaserverstärktem Kohlenstoff;
(3) Zusammenfügen der beiden Teile;
(4) Imprägnierung des Verbundes mit flüssigem Silizium. Dabei wird die Kappe aus polykristallinem, das heißt porösem Kohlenstoff, mit Silizium infiltriert, und es bildet sich der Siliziumcarbid-Kohlenstoff-Werkstoff aus. Außerdem entsteht an den Berührungsstellen zwischen der Kappe und dem Stift eine Siliziumcarbid-Verbindungsschicht 3, die die beiden Glieder der Prothese kraftschlüssig miteinander verbindet.

Beispiel 2

Bild 2 zeigt eine Schaftprothese für ein künstliches Hüftgelenk. Diese Prothese besitzt einen Femurkopf 1 aus einem Siliziumcarbid-Kohlenstoff-Werkstoff. Als Schaft 2 dient, wie in Beispiel 1, ein kohlenstoffaserverstärkter Kohlenstoff. Der Schaft hat am oberen Ende einen zylindrischen Zapfen von 10 mm ⌀, und die beiden Teile sind so gefertigt, daß zwischen dem Zapfen am oberen Ende des Schaftes und der Kugel eine Spaltbreite von 50 μm vorliegt.

Der Femurkopf war durch Imprägnieren eines vorgefertigten Teils aus polykristallinem (porösem) Kohlenstoff mit geschmolzenem Silizium hergestellt worden. Zur Herstellung der Verbindung 3 wurden die Verbindungsflächen beider Teile mit einer Aufschlämmung von Siliziumpulver in einem Gemisch aus einem in einem organischen Lösungsmittel gelösten, handelsüblichen Klebstoff und Aceton bestrichen. Danach wurden die Teile unmittelbar zusammengefügt, getrocknet und mit einer mittleren Aufheizrate von 300°C/h auf 1800°C aufgeheizt und bei dieser Temperatur 1 Std. gehalten. Anschließend wurde binnen 12 Std. auf Raumtemperatur abgekühlt.

**Patentansprüche**

1. Gelenkendoprothese mit zwei aufeinander gleitenden Prothesenteilen, bei der mindestens die Gleitfläche eines aus polykristallinem Kohlenstoff vorgefertigten Prothesenteils aus einem feinverteiltes Siliziumcarbid enthaltenden Kohlenstoffwerkstoff besteht, dadurch gekennzeichnet, daß der Siliziumcarbid enthaltende Kohlenstoffwerkstoff erhalten wurde durch Imprägnieren des vorgefertigten Prothesenteils mit geschmolzenem Silizium.

2. Gelenkendoprothese nach Anspruch 1, dadurch gekennzeichnet, daß der Siliziumcarbid-Kohlenstoff-Werkstoff aus 30 bis 70 Gew.-% Kohlenstoff und 70 bis 30 Gew.-% Siliziumcarbid besteht.

3. Gelenkendoprothese nach Anspruch 1 oder 2, dadurch gekennzeichnet, daß das andere Prothesenteil aus Siliziumcarbid-Kohlenstoff-Werkstoff besteht und ebenfalls durch Imprägnieren eines vorgefertigten Prothesenteils mit geschmolzenem Silizium erhalten wurde.

4. Gelenkendoprothese nach Anspruch 1 oder 2, dadurch gekennzeichnet, daß das andere Prothesenteil aus Kohlenstoff besteht.

5. Gelenkendoprothese nach einem der vorhergehenden Ansprüche, dadurch gekennzeichnet, daß das den Siliziumcarbid-Kohlenstoff-Werkstoff enthaltende Prothesenteil mehrere vorgefertigte formschlüssig sich berührende Glieder aus Kohlenstoffbasiswerkstoffen aufweist, die durch Behandlung mit Silizium unter Erwärmen an den Berührungsflächen kraftschlüssig miteinander verbunden worden sind.

6. Gelenkendoprothese nach Anspruch 5, dadurch gekennzeichnet, daß die vorgefertigten, formschlüssigen Prothesenglieder in einem Vakuum-Druck-Verfahren in einer flüssigen Siliziumschmelze unter Reaktion des Siliziums mit Kohlenstoff aus den Prothesengliedern behandelt worden sind, wobei an der Gleitfläche des Prothesenteils der Siliziumcarbid-Kohlenstoff-Werkstoff und zwischen den Gliedern eine Siliziumcarbid-Verbindungsschicht ausgebildet worden ist.

7. Gelenkendoprothese nach Anspruch 5, dadurch gekennzeichnet, daß die vorgefertigten, formschlüssigen Prothesenglieder, von denen mindestens ein Glied eine Gleitfläche aus mit geschmolzenem Silizium imprägniertem polykristallinem Kohlenstoff besitzt, an den Verbindungsflächen oberflächlich mit einer Aufschlämmung von Siliziumpulver bestrichen worden sind, die so behandelten Prothesenglieder zusammengefügt, getrocknet und zur Ausbildung einer Siliziumcarbid-Verbindungsschicht durch Reaktion des Siliziums mit Kohlenstoff aus den Prothesengliedern behandelt worden sind.

8. Gelenkendoprothese nach Anspruch 7, dadurch gekennzeichnet, daß die verwendete Aufschlämmung von Siliziumpulver zusätzlich Kohlenstoffpulver enthalten hat.

**Claims**

1. Articular endoprosthesis having two prosthetic parts sliding on one another, in which at least the sliding surface of one prosthetic part prefabricated from polycrystalline carbon consists of a carbon material containing finely divided silicon carbide, characterised in that the carbon material containing silicon carbide had been obtained by impregnating the prefabricated prosthetic part with molten silicon.

2. Articular entoprosthesis according to Claim 1, characterised in that the silicon carbide-carbon material consists of 30 to 70% by weight of carbon and 70 to 30% by weight of silicon carbide.

3. Articular endoprosthesis according to Claim 1 or 2, characterised in that the other prosthetic part consists of silicon carbide-carbon material and had likewise been obtained by impregnating a prefabricated prosthetic part with molten silicon.

4. Articular endoprosthesis according to Claim 1 or 2, characterised in that the other prosthetic part consists of carbon.

5. Articular endoprosthesis according to one of the preceding Claims, characterised in that the prosthetic part containing the silicon carbide-carbon material has a plurality of prefabricated members of carbon based materials, which in form-locking contact with one another have been connected together in force-locking manner at the contact surfaces by treatment with silicon while being heated.

6. Articular endoprosthesis according to Claim 5, characterised in that prefabricated form-locked prosthetic members have been treated in a vacuum-compression process in a liquid silicon smelt while reacting the silicon with carbon from the prosthetic members, there having been formed at the sliding surface of the prosthetic part the silicon carbide-carbon material and between the members a silicon carbide-connecting layer.

7. Articular endoprosthesis according to Claim 5, characterised in that the prefabricated form-locked prosthetic members, of which at least one member has a sliding surface of polycrystalline carbon impregnated with molten silicon, have been coated at the connecting surfaces superficially with a suspension of silicon powder, the thus treated prosthetic members have been joined together, dried and treated so as to form a silicon carbide-bonding layer by reaction of the silicon with carbon from the prosthetic members.

8. Articular prosthesis according to Claim 7, characterised in that the suspension of silicon powder used additionally contained carbon powder.

## Revendications

1. Endoprothèse d'articulation comportant deux parties de prothèse glissant l'une sur l'autre, dans laquelle au moins la surface de glissement d'une partie de prothèse préfabriquée en carbone polycristallin est formée d'une matière carbonée contenant du carbure de silicium finement divisé, caractérisée en ce que le matériau carboné contenant du carbure de silicium a été obtenu par imprégnation de la partie de prothèse préfabriquée avec du silicium fondu.

2. Endoprothèse d'articulation selon la revendication 1, caractérisée en ce que le matériau carbure de silicium-carbone est formé de 30 à 70% en poids de carbone et de 70 à 30% en poids de carbure de silicium.

3. Endoprothèse d'articulation selon l'une des revendications 1 ou 2, caractérisée en ce que l'autre partie de prothèse est formée de matériau carbure de silicium-carbone et a été également obtenue par imprégnation d'une partie de prothèse préfabriquée avec du silicium fondu.

4. Endoprothèse d'articulation selon l'une des revendications 1 ou 2, caractérisée en ce que l'autre partie de prothèse est formée de carbone.

5. Endoprothèse d'articulation selon l'une des revendications 1 à 4, caractérisée en ce que la partie de prothèse contenant le matériau carbure de silicium-carbone comporte plusieurs composants préfabriqués en matériaux à base de carbone qui sont en contact avec conjugaison de formes, lesdits composants ayant été liés entre eux avec conjugaison de forces sur les surfaces de contact par traitement avec du silicium après chauffage.

6. Endoprothèse d'articulation selon la revendication 5, caractérisée en ce que les composants de prothèse préfabriqués avec conjugaison de formes ont été traités selon un procédé faisant intervenir un vide et une pression, dans un bain de silicium liquide avec réaction du silicium sur le carbone provenant des composants de prothèse, une couche de liaison en carbure de silicium étant formée sur la surface de glissement de la partie de prothèse en matière carbure de silicium-carbone et entre les composants.

7. Endoprothèse d'articulation selon la revendication 5, caractérisée en ce que les composants de prothèse préfabriqués avec conjugaison de formes, parmi lesquels au moins un composant comporte une surface de glissement formée de carbone polycristallin imprégné de silicium fondu, ont été enduits superficiellement sur les surfaces de liaison d'une boue de poudre de silicium, les composants de prothèse ainsi traités ayant été assemblés, séchés et traités en vue de former une couche de liaison en carbure de silicium, par réaction du silicium avec du carbone provenant des composants de prothèse.

8. Endoprothèse d'articulation selon la revendication 7, caractérisée en ce que la boue de poudre de silicium utilisée contient additionnellement de la poudre de carbone.

Bild 1

Bild 2